# EUROPEAN PATENT SPECIFICATION

(11) **EP 1 731 138 B1**
(45) Date of publication and mention of the grant of the patent: **23.03.2016**
(21) Application number: 05727436.7
(22) Date of filing: 28.03.2005
(51) Int. Cl.: A61K 9/10, A61K 31/343, A61K 31/352, A61K 31/4709, A61K 31/497, A61K 31/7048, A61K 47/32, A61K 47/34, A61K 47/36

(54) **FINE DISPERSION OF SPARINGLY SOLUBLE DRUG AND PROCESS FOR PRODUCING THE SAME**
FEINE DISPERSION EINES SCHWERLÖSLICHEN ARZNEIMITTELS UND HERSTELLUNGSVERFAHREN DAFÜR
FINE DISPERSION DE MÉDICAMENT SOLUBLE ET PROCÉDÉ DE PRODUCTION DE CETTE DISPERSION

(30) Priority: 31.03.2004 JP 2004102780
(43) Date of publication of application: 13.12.2006
(73) Proprietor: TOYAMA CHEMICAL CO., LTD., Tokyo 160-0023 (JP)
(72) Inventor: KUBO, Yoshiko, 9393551 (JP); YAMAKAWA, Tetsumi, 9300036 (JP); YAMASAKI, Yasuomi, 9300816 (JP)
(74) Representative: Hartz, Nikolai
(86) International application number: PCT/JP2005/005736
(87) International publication number: WO 2005/094788

(56) References cited:
- EP-A2- 0 499 299
- WO-A1-2004/006959
- WO-A2-99/65469
- WO-A2-02/055059
- JP-A- 4 288 013
- JP-A- 2002 518 318
- US-A- 5 298 262
- US-A- 5 510 118
- BODMEIER R ET AL: "INDOMETHACIN POLYMERIC NANOSUSPENSIONS PREPARED BY MICROFLUIDIZATION", JOURNAL OF CONTROLLED RELEASE, ELSEVIER, AMSTERDAM, NL, vol. 12, no. 3, 1 May 1990 (1990-05-01), pages 223-233, XP000132663, ISSN: 0168-3659, DOI: 10.1016/0168-3659(90)90103-Z
- MUELLER R H ET AL: "Nanosuspensions as particulate drug formulations in therapy: Rationale for development and what we can expect for the future", ADVANCED DRUG DELIVERY REVIEWS, ELSEVIER BV, AMSTERDAM, NL, vol. 47, no. 1, 1 January 2001 (2001-01-01), pages 3-19, XP002232883, ISSN: 0169-409X, DOI: 10.1016/S0169-409X(00)00118-6

## Description

### TECHNICAL FIELD

The present invention relates to a fine dispersion of a poorly soluble drug and a process for producing the same. In more particular, the present invention relates to (1) a fine dispersion of a poorly soluble drug obtained by: suspending a poorly soluble drug in a liquid containing no deflocculant; introducing the resulting suspension into a high-pressure homogenizer to subject the same to a high-pressure treatment; and adding a deflocculant to the thus treated dispersion to deagglomerate aggregated particles contained therein, and (2) a process for producing the same.

### BACKGROUND ART

For a drug to show its activity, it needs to be in the dissolved form at its absorption site. However, a poorly soluble drug has a low dissolution rate, and in many cases, its dissolution process is rate-determining in its absorption. As methods for improving the solubility and absorption of a poorly soluble drug, there have been known: (1) a method in which a drug is micronized to fine particles; (2) a method in which a drug together with a polymer base are formed into a solid dispersion; (3) a method in which a drug together with any one of cyclodextrins are formed into a soluble complex; or (4) a method in which a drug is formed into an easily soluble salt.

It has been known that the absorption of a poorly soluble drug is improved by the above method (1), particularly when the poorly soluble drug is micronized to fine particles of which the particle size is less than 1000 nm (hereinafter referred to as particle size on the order of nanometer), due to its effects of: (1) increasing the surface area of the drug; (2) improving the solubility of the drug; (3) enhancing the dissolution rate of the drug; (4) increasing the adhesion of the drug particles to the surface of the mucosa at a site of absorption (Advanced Drug Delivery Reviews, vol. 47, 3 - 19, 2001).

Processes for micronizing a drug to fine particles are classified broadly into two categories: dry milling carried out in a gas; and wet milling carried out in a liquid atmosphere. Generally, by the dry milling, a drug is hard to be micronized to fine particles of size 10 µm or smaller, while by the wet milling, a drug can be micronized to fine particles of size less than 10 µm (Suguni Yakudatsu Ryushi Sekkei/Kakou Gijyutsu (Particulated Design and Pharmaceutical Technology), edit. by Jiho, Inc., 23 - 24, 2003). The wet milling is further classified into two major processes: mechanical grinding using a media mill etc.; and a pressure milling using a high-pressure homogenizer etc.

The mechanical grinding is to mill a material to be milled by: introducing milling media, such as balls or beads, and the material to be milled into a vessel; and rotating the vessel. For example, there has been known a process in which particles of a slightly soluble crystalline drug in the form of a mixed solution with a surface modifier is milled with a media mill (Japanese Patent No. 3602546). This milling, however, has disadvantages in that the drug as a material to be milled is susceptible to microbial contamination, because the drug in the form of a suspension undergoes grinding in the presence of a surface modifier usually for several days, and in that with the progress of milling, abrasion powders of the milling medium (balls or beads) and the milling vessel are contained into the milled drug (Advanced Drug Delivery Reviews, vol. 47, 3 - 19, 2001, Hunsai-Bunkyu to Hyomen Kaishitsu (Milling/Classification and Surface Modification), edit. by NGT, 75-76, 2001). For example, the iron limit of sodium chloride injection specified under USP28 (The United States Pharmacopeia 28^{th} edition) and that of water specified under the Japan Pharmacopoeia 14^{th} edition are 2 ppm and 0.3 mg/L, respectively. However, the content of the metal contamination in the drug milled by the above described method is larger than the above limits (Japanese Patent No. 3602546). Particularly in medicinal products such as injections, contamination of foreign bodies is a big problem from the viewpoint of product quality.

On the other hand, the pressure milling is to mill a material utilizing: the breaking action of the shear force produced when a pressurized liquid passes through a narrow nozzle or gap at high speed or of the impact force produced among the particles or the particles against the wall surface by the jet stream; or the shock wave produced by the cavitation. The pressure milling is suitable for production of medicinal products, because it uses no milling medium, and therefore, abrasion powder is less likely to be contaminated in the material to be milled (International Journal of Pharmaceutics, vol. 196, 169-172, 2000).

In the pressure milling, there has been known a process in which a poorly soluble drug is micronized to fine particles using a high-pressure homogenizer. A generally known process comprises a step of subjecting a liquid including dispersant and a poorly soluble drug to high-pressure treatment using a high-pressure homogenizer (Japanese Patent No. 2554784, Advanced Drug Delivery Reviews, vol. 47, 3 - 19, 2001), and fine dispersions of some drugs including paclitaxel and clofazimine in which the particles of the respective drugs of size on the order of nanometer are obtained. However, when the above described procedure is employed to treat a poorly soluble drug, the poorly soluble drug in the dispersion is hard to be micronized to fine particles of or smaller than a specified size, depending on the characteristics of the drug (Advanced Drug Delivery Reviews, vol. 47, 3 - 19, 2001). Further, when the above described procedure is employed to treat a poorly soluble drug, the particles size of the poorly soluble drug in the dispersion is likely to have a wide distribution and be non-uniform (International Journal of Pharmaceutics, vol. 214, 3-7, 2001) .

It is known that in a state where larger particles and smaller particles are mixed, a phenomenon occurs that the smaller particles are dissolved and disappear, while the larger particles grow more in size (Ostwald ripening) (Biryushi Kogaku Taikei (A Compendium of Fine Particle Engineering), vol. 1, Basic Technology, edit. by Fujitec, Corporation, 151-152, 2001). Fine particles of non-uniform particle size do not show fine-particle characteristics (improvement in solubility and dissolution rate, increase in adhesion, etc.) which should be stable with the passage of time.

In fine particles in a dispersion, the smaller the particle size becomes, the more vigorously their Brownian motion becomes; therefore, fine particles come close to and collide with each other more frequently. Thus, the effect of van der Waals force is made larger than the repulsive force among the particles, whereby the particles are more likely to aggregate. It is known that addition of a polymer has the effect of stabilizing the dispersion of fine particles. However, the dispersion stabilizing effect varies intricately depending on the molecular weight, molecular structure or content of the polymer added and the conditions under which the polymer is added. Particularly in fine particles having a particle size on the order of nanometer, aggregation occurs unless special repulsive action takes place among the particles (Biryushi Kogaku Taikei (A Compendium of Fine Particle Engineering), vol. 1, Basic Technology, edit. by Fujitec, Corporation, 238-242, 2001).

There has been known another process for producing a fine dispersion of particles of size on the order of nanometer in which a drug is dissolved in a water-miscible organic solvent and an aqueous solvent is added to the resulting solution to allow the drug to deposit (JP 2004-538249 A). This process is referred to as built-up process, which is clearly distinguished from the size-down process described in present invention. The process described in JP 2004-538249 A cannot be applied to drugs which are insoluble in a pharmaceutically allowable organic solvent.

Document US 5510118 A relates to a process for preparing therapeutic compositions containing nanoparticles.

### DISCLOSURE OF THE INVENTION

### PROBLEM TO BE SOLVED BY THE INVENTION

No process has been established for producing fine particles of a poorly soluble drug on the order of nanometer using a high-pressure homogenizer, and there have been strong needs for an effective and easy process for producing a fine dispersion of a poorly soluble drug and a fine dispersion of a poorly soluble drug having excellent dispersion stability. There have been also needs for medicinal preparations, as final products, containing lower content of contaminants.

### MEANS FOR SOLVING THE PROBLEM

In the circumstances, the present inventors directed tremendous research effort towards the solution of the above described problems, and they have finally found that a fine dispersion of a poorly soluble drug in which the particles of the poorly soluble drug micronized to fine particles of size on the order of nanometer are dispersed can be effectively and easily produced by: suspending a poorly soluble drug in a liquid containing no deflocculant to obtain a suspension; subjecting the suspension to high-pressure treatment using a high-pressure homogenizer to obtain a dispersion; and adding a deflocculant to the dispersion to deagglomerate aggregated particles contained therein. The term "deflocculant" used herein means an additive having the effect of dispersing aggregated particles.

In the first step of the process of the present invention, a poorly soluble drug is suspended in a liquid that contains no deflocculant to obtain a suspension, and the suspension is subjected to a high-pressure treatment using a high-pressure homogenizer to obtain a dispersion. Once the obtained dispersion is allowed to stand, the particles of the poorly soluble drug in the dispersion is aggregated and settled, and it looks as if the poorly soluble drug is not micronized to fine particles. However, in the second step, the aggregated particles of the poorly soluble drug are deagglomerated by: adding a deflocculant to the dispersion obtained in the first step; and, depending on the situation, subjecting the dispersion to deagglomeration treatment, such as a high-pressure treatment using a high-pressure homogenizer or ultrasonic treatment; as a result, surprisingly, a fine dispersion can be produced in which fine particles of the poorly soluble drug of size on the order of nanometer are dispersed. That is, when a poorly soluble drug is treated by a conventional method in common use, namely, a method in which a mixed liquid of a dispersant and a poorly soluble drug is treated using a high-pressure homogenizer, it is difficult to micronize the poorly soluble drug in the dispersion to fine particles of a specified size or smaller than the specific size depending on the characteristics of the drug; however, by the method of the present invention it is now possible to obtain a fine dispersion in which a poorly soluble drug is micronized to much finer particles.

Further, the inventors have found that by this method, the fine dispersion of a poorly soluble drug can be produced so that 90% by volume or more of the particles of the poorly soluble drug have a size less than 1000 nm or less than 500 nm in particle size, and besides, in the fine dispersion of a poorly soluble drug obtained by the present invention, the particle size distribution hardly changes with the passage of time during its storage and the particles of the poorly soluble drug has so excellent dispersion stability that they are never aggregated and settled even when being allowed to stand. Thus, they have accomplished the present invention.

### ADVANTAGE OF THE INVENTION

According to the process of the present invention, a fine dispersion of a poorly soluble drug can be easily produced in which the particles of the drug of size on the order of nanometer are dispersed and which has excellent dispersion stability over a long period of time. Further, the fine dispersion of a poorly soluble drug obtained by the production process of the present invention can be used to prepare an excellent medicinal preparation in which the content of contaminants is low.

### BEST MODE FOR CARRYING OUT THE INVENTION

In the following the present invention will be described in more detail.

The wording "poorly soluble drugs" used herein means "very slightly soluble" or "practically insoluble" drugs which are specified in the section under the heading Description, general notice, Japanese Pharmacopoeia 14^{th} edition. Specifically, they mean drugs of which the solubility in water at 20°C is lower than 1 mg/mL. Further specifically, they include: antibiotics such as cefditoren pivoxil, cefteram pivoxil, erythromycin, clarithromycin, telithromycin and azithromycin; pyridonecarboxylic acid synthetic antibacterial agents such as norfloxacin, tosufloxacin, sparfloxacin, nadifloxacin, enoxacin, cinoxacin, fleroxacin, prulifloxacin, nalidixic acid, pipemidic acid, piromidic acid and 1-cyclopropyl-8-methyl-7-[5-methyl-6-(methylamino)-3-pyridinyl]-4-oxo-1,4-dihydro-3-quinolinecarboxylic acid; antifungal agents, such as imidazole antifungal agents such as miconazole, clotrimazole and econazole nitrate, triazole antifungal agents such as itraconazole, polyene antifungal agents such as amphotericin B and the derivatives thereof, and griseofulvin; antiviral agents such as saquinavir, ritonavir, lopinavir, nevirapine, vidarabin, amprenavir and efavirenz; anti-inflammatory agents, such as propionic acid anti-inflammatory agents such as ibuprofen, ketoprofen and pranoprofen, arylacetic acid anti-inflammatory agents such as indomethacin, and oxicam anti-inflammatory agents such as piroxicam, ampiroxicam and lornoxicam; antirheumatic agents such as leflunomide, methotrexate, salazosulfapyridine, auranofin and iguratimod; antiallergic agents such as clemastine fumarate, loratadine, mequitazine, zafirlukast, pranlukast, ebastine, tazanolast, tranilast, ramatroban and oxatomide; gastrointestinal agents such as omeprazole, lansoprazol, teprenone, metoclopramide and sofalcone.

Examples of preferable poorly soluble drugs include: synthetic antibacterial agents, antifungal agents, antirheumatic agents, anti-inflammatory agents and gastrointestinal agents. More preferable are synthetic antibacterial agents, antifungal agents, antirheumatic agents, and much more preferable are synthetic antibacterial agents. Specific examples of preferable poorly soluble drugs include: 1-cyclopropyl-8-methyl-7-[5-methyl-6-(methylamino)-3-pyridinyl]-4-oxo-1,4-dihydro-3-quinolinecarboxylic acid, itraconazole, amphotericin B, griseofulvin and iguratimod. More preferable are iguratimod and 1-cyclopropyl-8-methyl-7-[5-methyl-6-(methylamino)-3-pyridinyl]-4-oxo-1,4-dihydro-3-quinolinecarboxylic acid, and much more preferable is 1-cyclopropyl-8-methyl-7-[5-methyl-6-(methylamino)-3-pyridinyl]-4-oxo-1,4-dihydro-3-quinolinecarboxylic acid.

As an antifungal agent, any one of imidazole antifungal agents, triazole antifungal agents, polyene antifungal agents and griseofulvin may be used. However, triazole antifungal agents and polyene antifungal agents are preferable and triazole antifungal agents are more preferable.

As a poorly soluble drug used in the present invention, preferable is a drug of which the solubility in water at 20°C is lower than 0.1 mg/mL and more preferably lower than 0.01 mg/mL.

The content of the poorly soluble drug in the dispersion used in the present invention is not limited to any specific one, as long as it can undergo high-pressure treatment using a high-pressure homogenizer. However, preferably, the content of the poorly soluble drug in the dispersion is 0.01 to 50% by weight and more preferably 0.01 to 30% by weight. When the fine dispersion of a poorly soluble drug is prepared into medicinal preparations in various dosage forms, the content can be adjusted to a desired one.

As a deflocculant used in the present invention, preferable is one or more polymers selected from natural polysaccharides and synthetic polymers.

Examples of natural polysaccharides used in the present invention include: acacia, xanthan gum and pullulan. Of these, acacia and pullulan are preferable.

Examples of synthetic polymers used in the present invention include: derivatives of natural polysaccharides, derivatives of vinyl polymer and copolymers of polyalkylene glycol.

Examples of derivatives of natural polysaccharides used in the present invention include: cellulose derivatives such as methylcellulose, hydroxypropylcellulose, hydroxypropylmethylcellulose, hydroxyethylcellulose and sodium carboxymethylcellulose; and starch derivatives such as hydroxypropylstarch. Of these, cellulose derivatives such as methylcellulose, hydroxypropylcellulose and hydroxypropylmethylcellulose are preferable.

Examples of derivatives of vinyl polymer used in the present invention include: polyvinyl alcohol, polyvinyl pyrrolidone and carboxyvinyl polymer. Of these, polyvinyl alcohol and polyvinyl pyrrolidone are preferable, and polyvinyl alcohol is more preferable.

As a copolymer of polyalkylene glycol used in the present invention, a polyoxyethylenepolyoxypropylene copolymer is preferable.

The concentration of the deflocculant used in the present invention is usually 0.001 to 20%, preferably 0.01 to 10% and more preferably 0.1 to 3% of the resultant fine dispersion, although it varies depending on the kind of the deflocculant and the method of adding the same. The deflocculant may be added to the dispersion obtained in the first step in the form of a solution prepared in advance by dissolving the deflocculant in a solvent or directly when the deflocculant is dissolved in the dispersion promptly. Or the deflocculant may be added, in the form of a solution prepared in advance by dissolving the deflocculant in a solvent, to the sediment containing the particles of the poorly soluble drug, which is obtained by centrifuging the dispersion obtained in the first step, or to the residue containing the particles of the poorly soluble drug, which is obtained by removing the solvent of the dispersion obtained in the first step under reduced pressure or the like.

Examples of liquids containing no deflocculant used in the first step of the present invention include: water, water-containing organic solvents, and organic solvents, more specifically, water, methanol, ethanol, propanol, 2-propanol, ethylene glycol, propylene glycol, glycerin, acetone, acetonitrile, dichloromethane and chloroform, and the mixed solutions thereof. Of these, preferable are water, ethanol, propanol, 2-propanol, ethylene glycol, propylene glycol, glycerin and acetone, and the mixed solutions thereof; and more preferable are water, ethanol, 2-propanol and acetone, and the mixed solutions thereof. Water is particularly preferable.

To the liquid containing no deflocculant used in the first step of the present invention, additives, other than a deflocculant, which do not prevent the poorly soluble drug from being micronized to fine particles may be added. Examples of such additives include: preservatives, isotonic agents, pH adjusters and buffers. Of these, pH adjusters and buffers are preferable.

Examples of preservatives used in the present invention include: paraoxybenzoic acid esters such as methyl paraoxybenzoate and ethyl paraoxybenzoate; such as benzyl alcohol, benzalkonium chloride, and benzethonium chloride.

Examples of isotonic agents used in the present invention include: sodium chloride, glucose, fructose, lactose, D-mannitol, D-sorbitol, xylitol and glycerin.

Examples of pH adjusters used in the present invention include: hydrochloric acid, acetic acid, sulfuric acid, phosphoric acid, methanesulfonic acid, lactic acid, oxalic acid, boric acid, citric acid, sodium hydroxide, potassium hydroxide, sodium hydrogencarbonate, monoethanolamine, diisopropanolamine, meglumine and trometamol.

Examples of buffers used in the present invention include: acids, bases, salts of acids and bases, and amino acids. These may be used in admixture thereof. Specific examples of such buffers include: mineral acids such as hydrochloric acid, nitric acid, phosphoric acid, sulfuric acid, boric acid and carbonic acid; organic carboxylic acids such as oxalic acid, citric acid, succinic acid, maleic acid, tartaric acid, lactic acid, acetic acid and benzoic acid; sulfonic acids such as methanesulfonic acid, benzenesulfonic acid and p-toluenesulfonic acid; inorganic bases such as sodium hydroxide, potassium hydroxide, magnesium hydroxide and calcium hydroxide; organic bases such as monoethanolamine, diethanolamine, triethanolamine, diisopropanolamine, meglumine and trometamol; salts of mineral acids and inorganic bases such as sodium chloride, trisodium phosphate, disodium hydrogenphosphate, sodium dihydrogenphosphate, tripotassium phosphate, dipotassium hydrogenphosphate, potassium dihydrogenphosphate, borax, sodium carbonate and sodium hydrogencarbonate; salts of organic carbolic acids and inorganic bases such as sodium citrate, sodium lactate, sodium acetate and sodium benzoate; salts of sulfonic aids and inorganic bases such as sodium methanesulfonate and sodium p-toluenesulfonate; aminosulfonic acids such as taurine; acidic amino acids such as aspartic acid and glutamic acid; neutral amino acids such as glutamine and glycine; and basic amino acids such as arginine and lysine.

In the present invention, when high-pressure treatment is performed using a high-pressure homogenizer, in order to retard the dissolution of the poorly soluble drug, formation of a solvate and change in crystal form, it is preferable to add any of the above described pH adjusters, buffers, etc. to the liquid containing no deflocculant used in the first step to adjust the pH, or the kind and/or concentration of ions of the liquid properly. Specific examples of methods for doing such adjustment include: when the poorly soluble drug is an zwitterionic electrolyte, such as 1-cyclopropyl-8-methyl-7-[5-methyl-6-(methylamino)-3-pyridinyl]-4-oxo-1,4-dihydro-3-quinolinecarboxylic acid, a method in which the pH of the liquid containing no deflocculant used in the first step is adjusted to pH close to the isoelectric point where the solubility of the drug is the lowest; when the poorly soluble drug is an acidic drug, a method in which the pH of the liquid containing no deflocculant used in the first step is lowered to a pH where ionization does not substantially occur; when the poorly soluble drug is a basic drug, a method in which the pH of the liquid is raised up to a pH where ionization does not substantially occur; when the poorly soluble drug is a basic drug, a method in which to the acid salt of the basic drug, the same kind of anions as the acid are added to shift the equilibrium, thereby lowering the solubility of the drug (common ion effect); and when the poorly soluble drug is capable of forming a solvate, a method in which a liquid which does not form a solvate is used as the liquid containing no deflocculant used in the first step. More specific examples of methods for doing such adjustment include: when the poorly soluble drug is a drug that forms a hydrate, such as iguratimod, a method in which a liquid other than water, such as ethanol, is used as the liquid containing no deflocculant used in the first step.

Any type of high-pressure homogenizer can be used in the present invention, as long as it can be used in the pressure milling. Examples of types of high-pressure homogenizers include: piston gap type, liquid jet mill type and high-pressure jet-stream reversing type of high-pressure homogenizers. Of these, the liquid jet mill type and high-pressure jet-stream reversing type are preferable, and the liquid jet mill type is more preferable. Specific examples of piston gap type of high-pressure homogenizers include: Manton Gaulin homogenizers (manufactured by APV). Specific examples of liquid jet mill type of high-pressure homogenizers include: Microfluidizer (manufactured by Mizuho Industry Co.,); Ultimizer (manufactured by SUGINO MACHINE) and Nanomizer (manufactured by YOSHIDA KIKAI CO., LTD.). Specific examples of high-pressure jet-stream reversing type of high-pressure homogenizers include: DeBEE (manufactured by NIPPON BEE).

The pressure at which the suspension of a poorly soluble drug in a liquid containing no deflocculant is subjected to high-pressure treatment using a high-pressure homogenizer in the first step of the present invention is preferably 100 MPa or higher, and more preferably 150 MPa to 300 MPa.

Examples of methods for subjecting the dispersion, after addition of a deflocculant, to deagglomeration treatment in the second step of the present invention include: a method in which the dispersion is subjected to high-pressure treatment using a high-pressure homogenizer; a method in which the dispersion is exposed to ultrasonic irradiation; and a method in which the dispersion is subjected to rotation treatment using a rotary homogenizer. Of these methods, a method in which the dispersion is subjected to high-pressure treatment using a high-pressure homogenizer and a method in which the dispersion is exposed to ultrasonic irradiation are preferable, and a method in which the dispersion is subjected to high-pressure treatment using a high-pressure homogenizer is more preferable.

In the second step of the present invention, the pressure at which the dispersion is subjected to high-pressure treatment using a high-pressure homogenizer is preferably 50 to 150 MPa.

In the present invention, when high-pressure treatment using a high-pressure homogenizer or ultrasonic treatment is performed, in order to retard the dissolution or decomposition of the poorly soluble drug, formation of a solvate and change in crystal form, it is preferable to control the temperature of the suspension and dispersion of a poorly soluble drug. Generally, high-pressure treatment and ultrasonic treatment involve temperature increase, thereby increasing the solubility of a drug; accordingly, it is preferably to cool the suspension of a poorly soluble drug with a coolant, depending on the situation, when high-pressure treatment or ultrasonic treatment is performed.

The wording "90% by volume or more of particles in the fine dispersion of a poorly soluble drug is less than 1000 nm in particle diameter" used herein means that 90% by volume or more of particles in the fine dispersion of a poorly soluble drug consists of fine particles less than 1000 nm in particle diameter, specifically it means the 90% cumulative diameter (D₉₀) of the volume distribution is less than 1000 nm in laser diffraction scattering particle size distribution measurement.

The wording "90% by volume or more of particles in the fine dispersion of a poorly soluble drug is less than 500 nm in particle diameter" used herein means that similar to the above, the 90% cumulative diameter (D₉₀) of the volume distribution is less than 500 nm.

The D₉₀ of a sparing soluble drug obtained in the present invention is preferably less than 1000 nm and more preferably less than 500 nm.

To the fine dispersion of a poorly soluble drug of the present invention, one or more additives, other than the above described preservatives, isotonic agents, pH adjusters and buffers, such as bioadhesive polymers and thickeners, may be added. With regard to the time when these additives are added, in one method, they are added when a deflocculant is added to the dispersion of a poorly soluble drug before treatment in the second step, and in another method, they are added after the second step is completed.

Examples of bioadhesive polymers adherent to living bodies used in the present invention include: chitosan and the derivatives thereof; and sodium hyaluronate.

Examples of thickeners used in the present invention include: dextran, carrageenan, alginic acid, sodium alginate and gellan gum.

The fine dispersion of a poorly soluble drug obtained by the present invention can be prepared into a medicinal preparation as necessary and administered in the dosage form of an external preparation, injection, oral preparation, inhalants or depot. Examples of external preparation include: eye drops, nasal drops, ear drops, solutions applied to the oral mucous membrane or skin, ointments, plaster, and suppositories applied to the anus or vagina. Examples of injections include: those for intravenous, intraarterial, intradermal, subcutaneous, intramuscular, intraarticular or intraorgan administration. Examples of oral preparations include: tablets, capsules, granules, powders, solutions and syrups. Examples of inhalants include: aerosols and powder inhaleres.

### EXAMPLES

In the following, the present invention will be described by Examples, Comparative Examples and Test Examples. It should not be understood that these examples are intended to limit the present invention. In the Examples and Comparative Examples, unless otherwise specified, the suspensions were cooled, during high-pressure treatment, in a double tube heat exchanger using water at about 10°C.

Iguratimod was prepared by the process described in Japanese Patent No. 2973143.
1-Cyclopropyl-8-methyl-7-[5-methyl-6-(methylamino)-3-pyridinyl]-4-oxo-1,4-dihydro-3-quinolinecarboxylic acid was prepared by the process described in WO02/062805.

### Example 1

3 g of 1-cyclopropyl-8-methyl-7-[5-methyl-6-(methylamino)-3-pyridinyl]-4-oxo-1,4-dihydro-3-quinolinecarboxylic acid (hereinafter referred to as T-3912) was suspended in 47 g of water. The suspension was subjected to high-pressure treatment at 200 MPa 300 cycles using a high-pressure homogenizer (Nanomizer, YSNM-2000AR, manufactured by YOSHIDA KIKAI CO., LTD.). To 31 g of resultant dispersion, 6 g of 6% aqueous solution of hydroxypropylmethylcellulose (Metholose 60SH-50, manufactured by Shin-Etsu Chemical Co., Ltd.) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) to yield 33 g of fine dispersion of T-3912.

### Example 2

3 g of T-3912 was suspended in 47 g of water. The suspension was subjected to high-pressure treatment at 200 MPa 300 cycles using a high-pressure homogenizer (Nanomizer, YSNM-2000AR, manufactured by YOSHIDA KIKAI CO., LTD.). To 3 g of resultant dispersion, 27 g of 2.2% aqueous solution of polyvinyl alcohol (Gosenol EG-25, manufactured by Nippon Synthetic Chemical Industry Co., Ltd.) was added, and the dispersion was subjected to high-pressure treatment at 100 MPa 10 cycles using a high-pressure homogenizer (Nanomizer, YSNM-1500, manufactured by YOSHIDA KIKAI CO., LTD.) to yield 30 g of fine dispersion of T-3912.

### Example 3

9 g of T-3912 was suspended in 141 g of water. The suspension was subjected to high-pressure treatment at 200 MPa 300 cycles using a high-pressure homogenizer (Nanomizer, YSNM-2000AR, manufactured by YOSHIDA KIKAI CO., LTD.). To 90 g of resultant dispersion, 18 g of 6% aqueous solution of hydroxypropylmethylcellulose (Metholose 60SH-50, manufactured by Shin-Etsu Chemical Co., Ltd.) was added, and the dispersion was subjected to high-pressure treatment at 150 MPa 10 cycles using a high-pressure homogenizer (Nanomizer, YSNM-2000AR, manufactured by YOSHIDA KIKAI CO., LTD.) to yield 68 g of fine dispersion of T-3912.

### Example 4

21 g of T-3912 was suspended in 329 g of water. The suspension was subjected to high-pressure treatment at 210 MPa 100 cycles using a high-pressure homogenizer (DeBEE, manufactured by NIPPON BEE). To 20 g of resultant dispersion, 180 g of 1.1% aqueous solution of hydroxypropylmethylcellulose (Metholose 60SH-50, manufactured by Shin-Etsu Chemical Co., Ltd.) was added, and the dispersion was subjected to high-pressure treatment at 100 MPa 10 cycles using a high-pressure homogenizer (DeBEE, manufactured by NIPPON BEE) to yield 200 g of fine dispersion of T-3912.

### Example 5

21 g of T-3912 was suspended in 329 g of water. The suspension was subjected to high-pressure treatment at 210 MPa 300 cycles using a high-pressure homogenizer (DeBEE, manufactured by NIPPON BEE). To 230 g of resultant dispersion, 46 g of 6% aqueous solution of hydroxypropylmethylcellulose (Metholose 60SH-50, manufactured by Shin-Etsu Chemical Co., Ltd.) was added, and the dispersion was subjected to high-pressure treatment at 100 MPa 20 cycles using a high-pressure homogenizer (DeBEE, manufactured by NIPPON BEE) to yield 250 g of fine dispersion of T-3912.

### Example 6

13 g of T-3912 was suspended in 247 g of water. The suspension was subjected to high-pressure treatment at 300 MPa 300 cycles using a high-pressure homogenizer (Ultimizer, manufactured by SUGINO MACHINE). To 220 g of resultant dispersion, 44 g of 6% aqueous solution of hydroxypropylmethylcellulose (Metholose 60SH-50, manufactured by Shin-Etsu Chemical Co., Ltd.) was added, and the dispersion was subjected to high-pressure treatment at 150 MPa 10 cycles using a high-pressure homogenizer (Ultimizer, manufactured by SUGINO MACHINE) to yield 230 g of fine dispersion of T-3912.

### Example 7

3 g of T-3912 was suspended in 47 g of water. The suspension was subjected to high-pressure treatment at 200 MPa 300 cycles using a high-pressure homogenizer (Nanomizer, YSNM-2000AR, manufactured by YOSHIDA KIKAI CO., LTD.). To 0.3 g of resultant dispersion, 4.7 g of 2.2% aqueous solution of acacia (trade name: JP acacia powder, Sanei Yakuhin Boeki KK.) was added, and the dispersion was subjected to ultrasonic irradiation for 10 minutes using an ultrasonic homogenizer (US-150, manufactured by NIHONSEIKI KAISHA LTD., tip diameter: 7 mm) to yield 5 g of fine dispersion of T-3912.

### Example 8

0.9 g of griseofulvin (manufactured by Wako Pure Chemical Industries, Ltd.) was suspended in 29.1 g of water. The suspension was subjected to high-pressure treatment at 200 MPa 300 cycles using a high-pressure homogenizer (Nanomizer, YSNM-2000AR, manufactured by YOSHIDA KIKAI CO., LTD.) (a time required: 1 hour and 10 minutes). The dispersion was cooled in a double tube heat exchanger during the high-pressure treatment. In the heat exchanger, a coolant (Naibrine, manufactured by Nisso Maruzen Chemical) cooled to 5°C with a low and constant-temperature water circulator (NCC-2100, manufactured by Tokyo Rikakikai Co., Ltd.) was circulated. To 20 g of resultant dispersion, 10 g of 6% aqueous solution of hydroxypropylmethylcellulose (Metholose 60SH-50, manufactured by Shin-Etsu Chemical Co., Ltd.) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) (a time required: 3 minutes) to yield 28.2 g of fine dispersion of griseofulvin.

### Example 9

0.9 g of iguratimod was suspended in 29.1 g of ethanol. The suspension was subjected to high-pressure treatment at 150 MPa 300 cycles using a high-pressure homogenizer (Nanomizer, YSNM-2000AR, manufactured by YOSHIDA KIKAI CO., LTD.) (a time required: 1 hour and 12 minutes). The dispersion was cooled during the high-pressure treatment with a coolant at 0°C in the same manner as in Example 8. To 25 g of resultant dispersion, 5 g of 6% aqueous solution of hydroxypropylmethylcellulose (HPC-L, manufactured by NIPPON SODA CO., LTD.) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) (a time required: 3 minutes) to yield 27.2 g of fine dispersion of iguratimod.

### Example 10

0.9 g of indomethacin (manufactured by Wako Pure Chemical Industries, Ltd.) was suspended in 29.1 g of water. The suspension was subjected to high-pressure treatment at 200 MPa 300 cycles using a high-pressure homogenizer (Nanomizer, YSNM-2000AR, manufactured by YOSHIDA KIKAI CO., LTD.) (a time required: 1 hour and 12 minutes). The dispersion was cooled during the high-pressure treatment with a coolant at 5°C in the same manner as in Example 8. To 25 g of resultant dispersion, 5 g of 6% aqueous solution of hydroxypropylmethylcellulose (Metholose 60SH-50, manufactured by Shin-Etsu Chemical Co., Ltd.) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) (a time required: 3 minutes) to yield 15.8 g of fine dispersion of indomethacin.

### Example 11

0.5 g of itraconazole (manufactured by LKT Laboratories) was suspended in 24.5 g of water. The suspension was subjected to high-pressure treatment at 200 MPa 300 cycles using a high-pressure homogenizer (Nanomizer, YSNM-2000AR, manufactured by YOSHIDA KIKAI CO., LTD.) (a time required: 1 hour and 38 minutes). The dispersion was cooled during the high-pressure treatment with a coolant at -5°C in the same manner as in Example 8. To 24.1 g of resultant dispersion, 2.7 g of 20% aqueous solution of acacia (Suzu Funmatsu Yakuhinn) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) (a time required: 4 minutes) to yield 24.8 g of fine dispersion of itraconazole.

### Example 12

0.6 g of amphotericin B (manufactured by Wako Pure Chemical Industries, Ltd.) was suspended in 29.4 g of water. The suspension was subjected to high-pressure treatment at 200 MPa 100 cycles using a high-pressure homogenizer (Nanomizer, YSNM-2000AR, manufactured by YOSHIDA KIKAI CO., LTD.) (a time required: 24 minutes). The dispersion was cooled during the high-pressure treatment with a coolant at 0°C in the same manner as in Example 8. To 23.5 g of resultant dispersion, 3.7 g of 15% aqueous solution of polyvinyl pyrrolidone (PLASDONE K-25, manufactured by ISP TECHNOLOGIES) was added, and the dispersion was subjected to high-pressure treatment at 100 MPa 10 cycles using a high-pressure homogenizer (Nanomizer, YSNM-2000AR, manufactured by YOSHIDA KIKAI CO., LTD.) (a time required: 3 minutes) to yield 28.3 g of fine dispersion of amphotericin B.

### Example 13

The same suspension of amphotericin B (manufactured by Wako Pure Chemical Industries, Ltd.) as prepared in Example 12 was subjected to high-pressure treatment at 200 MPa 100 cycles. To 6.5 g of resultant dispersion, 1 g of 15% aqueous solution of polyvinyl pyrrolidone (PLASDONE K-25, manufactured by ISP TECHNOLOGIES) was added, and the dispersion was subjected to ultrasonic irradiation for 10 minutes using an ultrasonic homogenizer (US-150, manufactured by NISSEI Corporation, tip diameter: 12 mm) to yield 7.5 g of fine dispersion of amphotericin B.

### Example 14

0.5 g of lansoprazol (manufactured by Toronto Research Chemicals) was suspended in 24.5 g of water. The suspension was subjected to high-pressure treatment, at 200 MPa 300 cycles using a high-pressure homogenizer (Nanomizer, YSNM-2000AR, manufactured by YOSHIDA KIKAI CO., LTD.) (a time required: 1 hour and 27 minutes). The dispersion was cooled during the high-pressure treatment with a coolant at -5°C in the same manner as in Example 8. To 0.3 g of resultant dispersion, 2 g of 2% aqueous solution of pullulan (HAYASHIBARA SHOJI, INC.) was added, and the dispersion was subjected to ultrasonic irradiation for 5 minutes using an ultrasonic homogenize (US-150, manufactured by NISSEI Corporation, tip diameter: 7 mm) to yield 2.3 g of fine dispersion of lansoprazol.

### Example 15

0.5 g of itraconazole (manufactured by LKT Laboratories) was suspended in 24.5 g of water. The suspension was subjected to high-pressure treatment at 200 MPa 600 cycles using a high-pressure homogenizer (Nanomizer, YSNM-2000AR, manufactured by YOSHIDA KIKAI CO., LTD.) (a time required: 3 hour and 3 minutes). The dispersion was cooled during the high-pressure treatment with a coolant at -5°C in the same manner as in Example 8. To 0.3 g of resultant dispersion, 2 g of 2% aqueous solution of acacia (Suzu Funmatsu Yakuhin) was added, and the dispersion was subjected to ultrasonic irradiation for 5 minutes using an ultrasonic homogenize (US-150, manufactured by NISSEI Corporation, tip diameter: 7 mm) to yield 2.3 g of fine dispersion of lansoprazol.

### Comparative Example 1

3 g of T-3912 was suspended in 47 g of 1% aqueous solution of hydroxypropylmethylcellulose (Metholose 60SH-50, manufactured by Shin-Etsu Chemical Co., Ltd.), and the suspension was subjected to high-pressure treatment at 200 MPa 300 cycles using a high-pressure homogenizer (Nanomizer, YSNM-2000AR, manufactured by YOSHIDA KIKAI CO., LTD.) to yield 36 g of fine dispersion of T-3912.

### Comparative Example 2

12.5 g of T-3912 was suspended in 237.5 g of 1% aqueous solution of hydroxypropylmethylcellulose (Metholose 60SH-50, manufactured by Shin-Etsu Chemical Co., Ltd.), and the suspension was subjected to high-pressure treatment at 210 MPa 100 cycles using a high-pressure homogenizer (DeBEE, manufactured by NIPPON BEE) to yield 245 g of fine dispersion of T-3912.

### Comparative Example 3

0.9 g of griseofulvin (manufactured by Wako Pure Chemical Industries, Ltd.) was suspended in 29.1 g of 2% aqueous solution of hydroxypropylmethylcellulose (Metholose 60SH-50, manufactured by Shin-Etsu Chemical Co., Ltd.), and the suspension was subjected to high-pressure treatment at 200 MPa 300 cycles, while cooling in the same manner as in Example 8, using a high-pressure homogenizer (Nanomizer, YSNM-2000AR, manufactured by YOSHIDA KIKAI CO., LTD.) to yield 27.0 g of fine dispersion of griseofulvin.

### Comparative Example 4

0.9 g of iguratimod was suspended in 29.1 g of 1% ethanol solution of hydroxypropylmethylcellulose (HPC-L, manufactured by NIPPON SODA CO., LTD.). The suspension was subjected to high-pressure treatment at 150 MPa 300 cycles, while cooling in the same manner as in Example 9, using a high-pressure homogenizer (Nanomizer, YSNM-2000AR, manufactured by YOSHIDA KIKAI CO., LTD.) (a time required: 1 hour and 33 minutes) to yield 25.1 g of fine dispersion of iguratimod.

### Comparative Example 5

0.9 g of indomethacin (manufactured by Wako Pure Chemical Industries, Ltd.) was suspended in 29.1 g of 1% aqueous solution of hydroxypropylmethylcellulose (Metholose 60SH-50, manufactured by Shin-Etsu Chemical Co., Ltd.). The suspension was subjected to high-pressure treatment at 200 MPa 300 cycles, while cooling in the same manner as in Example 10, using a high-pressure homogenizer (Nanomizer, YSNM-2000AR, manufactured by YOSHIDA KIKAI CO., LTD.) (a time required: 1 hour and 18 minutes) to yield 28.4 g of fine dispersion of indomethacin.

### Comparative Example 6

0.5 g of itraconazole (manufactured by LKT Laboratories) was suspended in 24.5 g of 2% aqueous solution of acacia (Suzu Funmatsu Yakuhinn). The suspension was subjected to high-pressure treatment at 200 MPa 300 cycles, while cooling in the same manner as in Example 11, using a high-pressure homogenizer (Nanomizer, YSNM-2000AR, manufactured by YOSHIDA KIKAI CO., LTD.) (a time required: 1 hour and 30 minutes) to yield 20.7 g of fine dispersion of itraconazole.

### Comparative Example 7

0.6 g of amphotericin B (manufactured by Wako Pure Chemical Industries, Ltd.) was suspended in 29.4 g of 2% aqueous solution of polyvinyl pyrrolidone (PLASDONE K-25, manufactured by ISP TECHNOLOGIES). The suspension was subjected to high-pressure treatment at 200 MPa 100 cycles, while cooling in the same manner as in Example 12, using a high-pressure homogenizer (Nanomizer, YSNM-2000AR, manufactured by YOSHIDA KIKAI CO., LTD.) (a time required: 24 minutes) to yield 30.0 g of fine dispersion of amphotericin B.

### Comparative Example 8

0.5 g of lansoprazol (manufactured by Toronto Research Chemicals) was suspended in 24.5 g of 2% aqueous solution of pullulan (HAYASHIBARA SHOJI, INC.). The suspension was subjected to high-pressure treatment at 200 MPa 300 cycles, while cooling in the same manner as in Example 14, using a high-pressure homogenizer (Nanomizer, YSNM-2000AR, manufactured by YOSHIDA KIKAI CO., LTD.) (a time required: 1 hour and 21 minutes) to yield 10.3 g of fine dispersion of lansoprazol.

### Comparative Example 9

0.6 g of amphotericin B (manufactured by Wako Pure Chemical Industries, Ltd.) was suspended in 29.4 g of water. The suspension was subjected to high-pressure treatment at 200 MPa 300 cycles, while cooling in the same manner as in Example 12, using a high-pressure homogenizer (Nanomizer, YSNM-2000AR, manufactured by YOSHIDA KIKAI CO., LTD.) (a time required: 1 hour and 9 minutes) to yield 28.3 g of fine dispersion of amphotericin B.

### Test Example 1 - Particle size measurement

The particle size distribution in each of the fine dispersions of Examples and Comparative Examples was measured using a laser diffraction particle size analyzer (LS 13 320, manufactured by Beckman Coulter K.K.) or laser diffraction particle size analyzer (LA-920, manufactured by HORIBA).

The 50% cumulative diameter and 90% cumulative diameter of each of Comparative Example 1 and Example 1 are shown in Table 1.

Table 1 shows that the particle size of the particles of Example 1 was less than that of Comparative Example 1. Further, Table 1 shows that the particle size distribution in the dispersion of Comparative Example 1 was wide and the 50% cumulative diameter of the same was larger than 1000 nm, which indicates that the percentage of the fine particles of which the particle size was less than 1000 nm was 50% or lower. In contrast, the particle size distribution of the particles of Example 1 was narrow and the 90% cumulative diameter of the same was less than 1000 nm, which indicates that 90% or more of the particles of the dispersion of Example 1 was fine particles of size on the order of nanometer less than 1000 nm.

**[Table 1]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Comparative Example 1 | Nanomizer | 200 | 300 | 1443 | 4810 |
| Example 1 | Nanomizer | 200 | 300 | 205 | 374 |

The 50% cumulative diameter and 90% cumulative diameter of each of Comparative Example 2 and Example 4 are shown in Table 2.
Table 2 confirms that the 50% cumulative diameter and the 90% cumulative diameter of the particles of Example 4 were less than those of Comparative Example 2 and that 90% or more of the particles of Example 4 were fine particles of size on the order of nanometer less than 1000 nm.

**[Table 2]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Comparative Example 2 | DeBEE | 210 | 100 | 672 | 1874 |
| Example 4 | DeBEE | 210 | 100 | 322 | 753 |

The 50% cumulative diameter and 90% cumulative diameter of each of Examples 2, 3, 5, 6 are shown in Table 3.

Table 3 confirms that 90% or more of the particles of Examples 2, 3, 5, 6 were fine particles of size on the order of nanometer less than 1000 nm.

**[Table 3]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Example 2 | Nanomizer | 200 | 300 | 74 | 255 |
| Example 3 | Nanomizer | 200 | 300 | 113 | 239 |
| Example 5 | DeBEE | 210 | 300 | 261 | 381 |
| Example 6 | Ultimizer | 300 | 300 | 265 | 382 |

### Test Example 2 - Evaluation of dispersion stability of fine dispersions of T-3912 by appearances

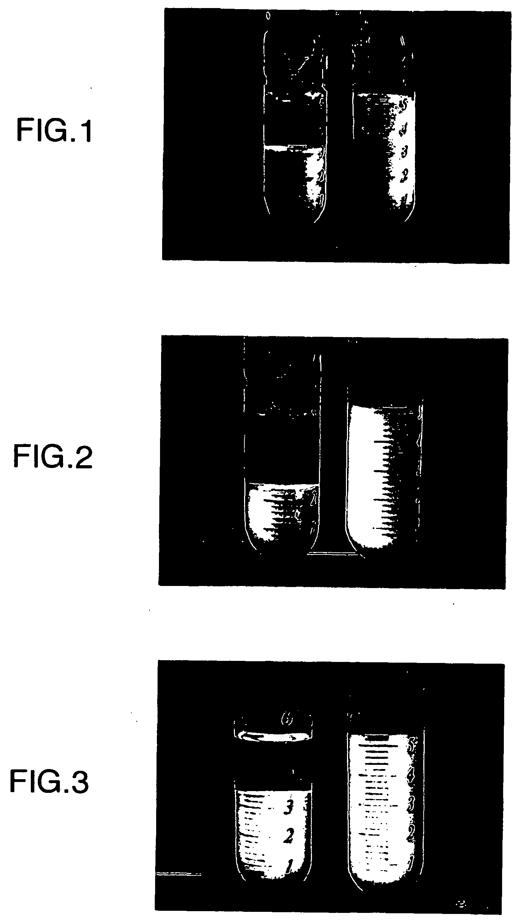

(1) The appearance of the fine dispersions of Comparative Example 1 and Example 1 was observed after allowing the dispersions to stand, while keeping in cold storage, for 8 weeks. The result is shown in Fig. 1. The observation confirmed that the fine dispersion of Comparative Example 1 (in the left test tube of Fig. 1) had a clear layer in its upper part and sedimentary particles, while the entire fine dispersion of Example 1 (in the right test tube of Fig. 1) was kept white turbidity.
(2) The appearance of the fine dispersions of Comparative Example 1 and Example 1 was observed after allowing the dispersions to stand, while keeping in cold storage, for 14 months. The result is shown in Fig. 2. The observation confirmed that the fine dispersion of Comparative Example 1 (in the left test tube of Fig. 2) had a clear layer in its upper part and sedimentary particles, while the entire fine dispersion of Example 1 (in the right test tube of Fig. 2) was kept white turbidity.

### Test Example 3 - Evaluation of dispersion stability of fine dispersion of T-3912 by particle size

The particle size distribution of the fine dispersion of Example 3 was measured immediately after the preparation and 4 months after the preparation. The results are shown in Table 4. No change was observed in the particle size distribution between immediately after the preparation and even after 4 months. The measurement confirmed that the fine dispersion of Example 3 was kept stable even after 4 months had elapsed.

In other words, the measurement confirmed that the fine dispersion of a poorly soluble drug of the present invention hardly suffered changes in particle size distribution with the passage of time during its storage and had excellent dispersion stability.

**[Table 4]**

| | 50% cumulative diameter [nm] | 90% cumulative diameter [nm] |
|---|---|---|
| Immediately after preparation | 113 | 239 |
| 4 months after preparation | 108 | 248 |

### Test Example 4 - Evaluation of dispersion stability of fine dispersions of T-3912 by particle size

The particle size distribution of the fine dispersions of Comparative Example 1 and Example 1 was measured immediately after the preparation and 12 months after the preparation. The results are shown in Table 5. In the dispersion of Comparative Example 1, the 50% cumulative diameter and 90% cumulative diameter were larger at 12 months after the preparation than immediately after the preparation, while in the dispersion of Example 1, no change was observed in the particle size distribution between immediately after the preparation and 12 months after the preparation. The measurement confirmed that the fine dispersion of Example 1 was kept stable even after 12 months had elapsed.

In other words, the measurement confirmed that the fine dispersion of a poorly soluble drug of the present invention hardly suffered changes in particle size distribution with the passage of time during its storage and had excellent dispersion stability.

**[Table 5]**

| | Time | 50% cumulative diameter [nm] | 90% cumulative diameter [nm] |
|---|---|---|---|
| Comparative Example 1 | Immediately after preparation | 1443 | 4810 |
| | 12 months after preparation | 3624 | 7289 |
| Example 1 | Immediately after preparation | 205 | 374 |
| | 12 months after preparation | 185 | 337 |

### Test Example 5 - Measurement of particle size

The 50% cumulative diameter and 90% cumulative diameter of the particles of each of Comparative Example 3 and Example 8, along with the 50% cumulative diameter and 90% cumulative diameter of each of the particles of Examples 9 to 14 and the corresponding Comparative Examples are shown in Table 6.

Table 6 shows that the 50% cumulative diameter and 90% cumulative diameter of the particles of Example 8 were less than those of Comparative Example 3, and the measurement confirmed that 90% or more of the particles of Example 8 were fine particles of size on the order of nanometer less than 1000 nm. In the other comparative experiments, the 50% cumulative diameter and 90% cumulative diameter of the particles of Examples were less than those of the corresponding Comparative Examples, and the measurement confirmed that 90% or more of the particles were fine particles of size on the order of nanometer less than 1000 nm in the dispersions of all Examples.

**[Table 6]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Comparative Example 3 | Nanomizer | 200 | 300 | 4157 | 7223 |
| Example 8 | Nanomizer | 200 | 300 | 422 | 784 |
| Comparative Example 4 | Nanomizer | 150 | 300 | 362 | 1693 |
| Example 9 | Nanomizer | 150 | 300 | 313 | 567 |
| Comparative Example 5 | Nanomizer | 200 | 300 | 421 | 882 |
| Example 10 | Nanomizer | 200 | 300 | 274 | 455 |
| Comparative Example 6 | Nanomizer | 200 | 300 | 570 | 1510 |
| Example 11 | Nanomizer | 200 | 300 | 431 | 942 |
| Comparative Example 7 | Nanomizer | 200 | 100 | 256 | 1224 |
| Example 12 | Nanomizer | 200 | 100 | 210 | 633 |
| Example 13 | Nanomizer | 200 | 100 | 208 | 633 |
| Comparative Example 8 | Nanomizer | 200 | 300 | 4829 | 12780 |
| Example 14 | Nanomizer | 200 | 300 | 225 | 349 |

The 50% cumulative diameter and 90% cumulative diameter of particles of Example 15 are shown in Table 7. Table 7 confirms that 90% or more of the particles of Example 15 were fine particles of size on the order of nanometer less than 1000 nm.

**[Table 7]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Example 15 | Nanomizer | 200 | 600 | 316 | 595 |

The 50% cumulative diameter and 90% cumulative diameter of particles of Comparative Example 9 are shown in Table 8. Table 8 confirms that when no deflocculant was added, both the 50% cumulative diameter and 90% cumulative diameter were 1000 nm or larger and the particles of size on the order of nanometer were not obtained.

**[Table 8]**

| | Name of high-pressure homogenizer | Treatment pressure [MPa] | Number of treatment [Cycle] | 50% cumulative diameter [nm] | 90% cumulative diameter [nm] |
|---|---|---|---|---|---|
| Comparative Example 9 | Nanomizer | 200 | 300 | 1007 | 1829 |

### Test Example 6 - Evaluation of dispersion stability of fine dispersions of griseofulvin by appearances

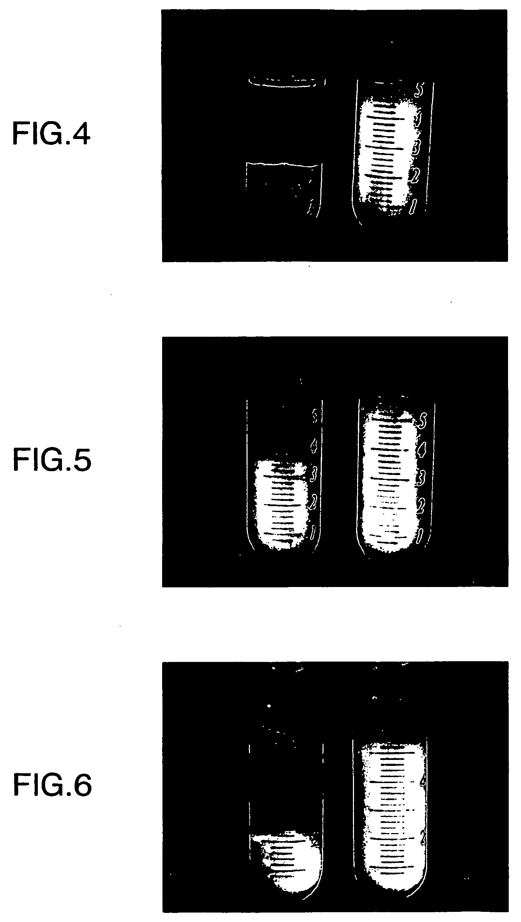

The appearance of the fine dispersions of Comparative Example 3 and Example 8 was observed after allowing the dispersions to stand, while keeping in cold storage, for 24 hours. The result is shown in Fig. 3. Similarly, the appearance of the fine dispersions of Comparative Example 3 and Example 8 was observed after allowing the dispersions to stand, while keeping in cold storage, for 9 weeks. The result is shown in Fig. 4. The observation confirmed that the fine dispersion of Comparative Example 3 (in the left test tube of Figs. 3, 4) had a clear layer in its upper part and sedimentary particles after 24 hours and the clear layer became larger after 9 weeks, while the entire fine dispersion of Example 8 (in the right test tube of Fig. 3, 4) was kept white turbidity even after 9 weeks.

### Test Example 7 - Evaluation of dispersion stability of fine dispersions of itraconazole by appearances

The appearance of the fine dispersions of Comparative Example 6 and Example 11 was observed after allowing the dispersions to stand, while keeping in cold storage, for 24 hours. The result is shown in Fig. 5. Similarly, the appearance of the fine dispersions of Comparative Example 6 and Example 11 was observed after allowing the dispersions to stand, while keeping in cold storage, for 4 weeks. The result is shown in Fig. 6. The observation confirmed that the fine dispersion of Comparative Example 6 (in the left test tube of Fig. 5) had a clear layer in its upper part after 24 hours and the clear layer became larger and the sedimentation progresses during 4 weeks, while the entire fine dispersion of Example 11 (in the right test tube of Figs. 5, 6) was kept white turbidity even after 4 weeks.

### Test Example 8 - Evaluation of dispersion stability of fine dispersions of amphotericin B by appearances

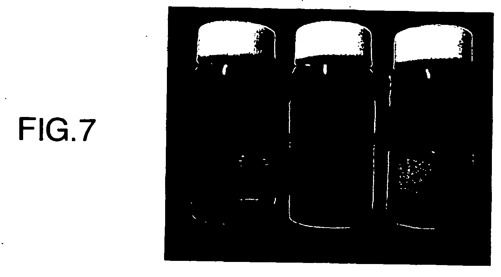

The appearance of the fine dispersions of Comparative Examples 7, 9 and Example 12 was observed after allowing the dispersions to stand, while keeping in cold storage, for 9 weeks. The result is shown in Fig. 7. The observation confirmed that the fine dispersion of Comparative Examples 9 (in the left screw capped tube of Fig. 7) and that of Comparative Example 7 (in the mid screw capped tube of Fig. 7) had a clear layer in their upper part and sedimentary particles after 9 weeks, while the entire fine dispersion of Example 8 (in the right screw capped tube of Fig. 7) was kept in the light yellow suspension state even after 9 weeks.

### Test Example 9 - Determination of metal concentration of fine dispersion of T-3912

Concentrated sulfuric acid and concentrated nitric acid were added to the fine dispersions obtained in Examples 3 and 6, followed by heating, and T-3912 and hydroxypropylmethylcellulose were subjected to ashing by the conventional procedure. The metal concentration of each of the fine dispersions was determined using an ICP emission spectrochemical analyzer (SPS3000 ICP, manufactured by Seiko Instruments Inc.). The major material for the portion of a high-pressure homogenizer which comes in contact with dispersions is stainless steel. The measurements of the concentration of iron, which is the major constituent of stainless steel, are shown in Table 9. In both fine dispersions of Example 3 and Example 6, the iron concentration was lower than 1 ppm, which confirmed that iron contamination resulting from high-pressure treatment was very small.

In both fine dispersions, the concentrations of harmful heavy metals, such as lead, bismuth, copper, cadmium, antimony, tin and mercury, were 1 ppm or lower and the concentrations of other metals, such as chromium, nickel, cobalt, zinc, tungsten, aluminum, silicon, manganese, molybdenum and zirconium, were 5 ppm or lower.

Specifically, the fine dispersions of a poorly soluble drug of the present invention hardly cause low metal contamination, and thus, they are suitably used for production of medicinal preparations.

**[Table 9]**

| | Name of high-pressure homogenizer | Treatment pressure [MPa] | Number of treatment [Cycle] | Iron concentration [ppm] |
|---|---|---|---|---|
| Example 3 | Nanomizer | 200 | 300 | 0.8 |
| Example 5 | Ultimizer | 300 | 300 | 0.5 |

### Preparation Example 1 - Eye drop

The fine dispersion of T-3912 prepared in the same manner as in Example 5 was centrifuged, and the supernatant was passed through a membrane filter with a pore diameter of 0.2 microns (Posidyne, manufactured by Pall Corporation). To 2 g of the filtrate, 2 g of 2% aqueous solution of chitosan (PROTOSAN G-213, manufactured by PRONOVA) was added. The mixed solution was subjected to isotonicity by adding glycerin (manufactured by Maruishi Pharmaceutical Co., Ltd.), whereby an eye drop was prepared.

### Preparation Example 2 - External preparation

To 2.5 g of the fine dispersion of iguratimod prepared in Example 9, 2.25 g of propylene glycol (manufactured by Maruishi Pharmaceutical Co., Ltd.) and 0.25 g of isopropyl myristate (IPM-100, manufactured by Nippon Surfactant Kogyo K.K.) were added so as to prepare an external preparation of iguratimod.

### Preparation Example 3 - Injection

To 1 g of the fine dispersion of itraconazole prepared in Example 11, 0.009 g of sodium chloride (manufactured by Wako Pure Chemical Industries, Ltd.) was added and dissolved. The dispersion was subjected to autoclaving (autoclave, SS-320, manufactured by TOMY SEIKO Co., Ltd.) to prepare an injection of itraconazole.

### Preparation Example 4 - Syrup

To 1 g of the fine dispersion of amphotericin B prepared in Example 12, 1 g of glycerin (manufactured by Maruishi Pharmaceutical Co., Ltd.) and 8 g of simple syrup (manufactured by Maruishi Pharmaceutical Co., Ltd.) were added to prepare a syrup of amphotericin B.

### Preparation Example 5 - Granule

1.75 g of the fine dispersion of iguratimod prepared in Example 9 was added to 1 g of lactose (Pharmatose 50M, manufactured by DMV) heated to 40°C, and the mixture was granulated and dried to prepare a granule of iguratimod.

### INDUSTRIAL APPLICABILITY

According to the production process of the present invention, a fine dispersion of a poorly soluble drug can be easily produced in which the fine particles of the drug of size on the order of nanometer are dispersed and which has excellent dispersion stability over a long period of time. Further, the fine dispersion of a poorly soluble drug obtained by the production process of the present invention can be used to prepare an excellent medicinal preparation in which the content of contaminants is low.

### BRIEF DESCRIPTION OF THE DRAWINGS

Fig. 1 is a photograph of the appearance of the fine sparingly-soluble-drug dispersions of Comparative Example 1 and Example 1 after allowing to stand, while keeping in cold storage, for 8 weeks;
Fig. 2 is a photograph of the appearance of the fine sparingly-soluble-drug dispersions of Comparative Example 1 and Example 1 after allowing to stand, while keeping in cold storage, for 14 months;
Fig. 3 is a photograph of the appearance of the fine sparingly-soluble-drug dispersions of Comparative Example 3 and Example 8 after allowing to stand, while keeping in cold storage, for 24 hours;
Fig. 4 is a photograph of the appearance of the fine sparingly-soluble-drug dispersions of Comparative Example 3 and Example 8 after allowing to stand, while keeping in cold storage, for 9 weeks;
Fig. 5 is a photograph of the appearance of the fine sparingly-soluble-drug dispersions of Comparative Example 6 and Example 11 after allowing to stand, while keeping in cold storage, for 24 hours;
Fig. 6 is a photograph of the appearance of the fine sparingly-soluble-drug dispersions of Comparative Example 6 and Example 11 after allowing to stand, while keeping in cold storage, for 4 weeks; and
Fig. 7 is a photograph of the appearance of the fine sparingly-soluble-drug dispersions of Comparative Example 7, Comparative Example 9 and Example 12 after allowing to stand, while keeping in cold storage, for 9 weeks.

## Claims

1. A process for producing a fine dispersion of a poorly soluble drug, **characterized by** comprising the steps of:
suspending a poorly soluble drug in a liquid containing no deflocculant to obtain a suspension; introducing the suspension into a high-pressure homogenizer, followed by subjecting the suspension to high-pressure treatment to obtain a dispersion; and adding a deflocculant to the dispersion to deagglomerate aggregated particles contained therein;
wherein
the poorly soluble drug is a drug having a solubility of lower than 1 mg/mL in water at 20°C;
the deflocculant is a synthetic polymer or a natural polysaccharide;
90% by volume or more of particles in the fine dispersion is less than 1000 nm in particle diameter; and
the high-pressure means 100 MPa or higher.

2. The process according to Claim 1, wherein the synthetic polymer is a natural polysaccharide derivative, a vinyl polymer derivative or a copolymer of polyalkylene glycol.

3. The process according to any one of Claims 1 to 2, wherein the poorly soluble drug is a synthetic antibacterial agent, antifungal agent, antirheumatic agent, anti-inflammatory agent or gastrointestinal agent.

4. The process according to any one of Claims 1 to 2, wherein the poorly soluble drug is a synthetic antibacterial agent, antirheumatic agent or antifungal agent.

5. The process according to any one of Claims 3 to 4, wherein the antifungal agent is a triazole antifungal agent or polyene antifungal agent.

6. The process according to any one of Claims 1 to 2, wherein the poorly soluble drug is a synthetic antibacterial agent.

7. The process according to any one of Claims 1 to 2, wherein the poorly soluble drug is 1-cyclopropyl-8-methyl-7-[5-methyl-6-(methylamino)-3-pyridinyl]-4-oxo-1,4-dihydro-3-quinolinecarboxylic acid, itraconazole, amphotericin B, griseofulvin or iguratimod.

8. The process according to any one of Claims 1 to 2, wherein the poorly soluble drug is iguratimod.

9. The process according to any one of Claims 1 to 2, wherein the poorly soluble drug is 1-cyclopropyl-8-methyl-7-(5-methyl-6-(methylamino)-3-pyridinyl]-4-oxo-1,4-dihydro-3-quinolinecarboxylic acid.

10. The process according to any one of Claims 1 to 6, wherein the poorly soluble drug is a drug having a solubility in water at 20°C of lower than 0.1 mg/mL.

## Patentansprüche

1. Verfahren zum Herstellen einer feinen Dispersion eines schwer löslichen Medikaments, **dadurch gekennzeichnet, dass** das Verfahren folgende Schritte umfasst:
Suspendieren eines schwer löslichen Medikaments in einer kein Entflockungsmittel enthaltenden Flüssigkeit, zum Erhalt einer Suspension;
Einleiten der Suspension in einen Hochdruck-Homogenisator, gefolgt von Unterziehen der Suspension einer Hochdruck-Behandlung, zum Erhalt einer Dispersion; und Zugabe eines Entflockungsmittels zu der Dispersion zum Deagglomerieren der in derselben enthaltenen aggregierten Partikel;
wobei das schwer lösliche Medikament ein Medikament mit einer Löslichkeit in Wasser bei 20 °C von weniger als 1 mg/l ist;
das Entflockungsmittel ein synthetisches Polymer oder ein natürliches Polysaccharid ist;
90 Vol.-% oder mehr der Partikel in der feinen Dispersion einen Partikeldurchmesser kleiner als 1000 nm besitzen; und
Hochdruck 100 MPa oder höher bedeutet.

2. Verfahren nach Anspruch 1, wobei das synthetische Polymer ein Derivat eines natürlichen Polysaccharids ist, ein Vinylpolymer-Derivat oder ein Copolymer von Polyalkylenglycol.

3. Verfahren nach einem der Ansprüche 1 bis 2, wobei das schwerlösliche Medikament ein synthetisches antibakterielles Mittel ist, ein Antimykotikum, ein Antirheumatikum, ein Antiphlogistikum oder ein gastrointestinales Mittel.

4. Verfahren nach einem der Ansprüche 1 bis 2, wobei das schwerlösliche Medikament ein synthetisches antibakterielles Mittel, ein Antirheumatikum oder ein Antimykotikum ist.

5. Verfahren nach einem der Ansprüche 3 bis 4, wobei das Antimykotikum ein Triazol-Antimykotikum oder ein Polyen-Antimykotikum ist.

6. Verfahren nach einem der Ansprüche 1 bis 2, wobei das schwerlösliche Medikament ein synthetisches antibakterielles Mittel ist.

7. Verfahren nach einem der Ansprüche 1 bis 2, wobei das schwerlösliche Medikament 1-Cyclopropyl-8-methyl-7-[5-methyl-6-(methylamino)-3-pyridinyl]-4-oxo-1,4-dihydro-3-chinolincarbonsäure, Itraconazol, Amphotericin B, Griseofulvin oder Iguratimod ist.

8. Verfahren nach einem der Ansprüche 1 bis 2, wobei das schwerlösliche Medikament Iguratimod ist.

9. Verfahren nach einem der Ansprüche 1 bis 2, wobei das schwerlösliche Medikament 1-Cyclopropyl-8-methyl-7-[5-methyl-6-(methylamino)-3-pyridinyl]-4-oxo-1,4-dihydro-3-chinolincarbonsäure ist.

10. Verfahren nach einem der Ansprüche 1 bis 6, wobei das schwerlösliche Medikament ein Medikament mit einer Löslichkeit in Wasser bei 20 °C von weniger als 0,1 mg/ml ist.

## Revendications

1. Procédé pour produire une dispersion fine d'un médicament médiocrement soluble, **caractérisé en ce qu'**il comprend les étapes de : mise en suspension d'un médicament médiocrement soluble dans un liquide ne contenant pas de défloculant pour former une suspension ; introduction de la suspension dans un homogénéisateur haute pression, suivie de la soumission de la suspension à un traitement sous haute pression pour former une dispersion ; et addition d'un défloculant à la dispersion pour désagglomérer les particules agrégées qu'elle contient ;
dans lequel
le médicament médiocrement soluble est un médicament présentant une solubilité inférieure à 1 mg/ml dans l'eau à 20°C ;
le défloculant est un polymère synthétique ou un polysaccharide naturel ;
90 % en volume ou plus des particules dans la dispersion fine ont une granulométrie inférieure à 1000 nm ; et
la haute pression signifie 100 MPa ou plus.

2. Procédé selon la revendication 1, dans lequel le polymère synthétique est un dérivé de polysaccharide naturel, un dérivé de polymère de vinyle ou un copolymère de polyalkylèneglycol.

3. Procédé selon l'une quelconque des revendications 1 et 2, dans lequel le médicament médiocrement soluble est un agent antibactérien synthétique, un agent antifongique, un agent antirhumatismal, un agent anti-inflammatoire ou un agent gastro-intestinal.

4. Procédé selon l'une quelconque des revendications 1 et 2, dans lequel le médicament médiocrement soluble est un agent antibactérien synthétique, un agent antirhumatismal ou un agent antifongique.

5. Procédé selon l'une quelconque des revendications 3 et 4, dans lequel l'agent antifongique est un agent antifongique de type triazole ou un agent antifongique de type polyène.

6. Procédé selon l'une quelconque des revendications 1 et 2, dans lequel le médicament médiocrement soluble est un agent antibactérien synthétique.

7. Procédé selon l'une quelconque des revendications 1 et 2, dans lequel le médicament médiocrement soluble est l'acide 1-cyclopropyl-8-méthyl-7-[5-méthyl-6-(méthylamino)-3-pyridinyl]-4-oxo-1,4-dihydro-3-quinolinecarboxylique, l'itraconazole, l'amphotéricine B, la griséofulvine ou l'iguratimod.

8. Procédé selon l'une quelconque des revendications 1 et 2, dans lequel le médicament médiocrement soluble est l'iguratimod.

9. Procédé selon l'une quelconque des revendications 1 et 2, dans lequel le médicament médiocrement soluble est l'acide 1-cyclopropyl-8-méthyl-7-[5-méthyl-6-(méthylamino)-3-pyridinyl]-4-oxo-1,4-dihydro-3-quinolinecarboxylique.

10. Procédé selon l'une quelconque des revendications 1 à 6, dans lequel le médicament médiocrement soluble est un médicament présentant une solubilité dans l'eau à 20°C inférieure à 0,1 mg/ml.
